# NEUE EUROPÄISCHE PATENTSCHRIFT

(11) **EP 3 694 351 B2**
(45) Veröffentlichungstag und Bekanntmachung des Hinweises auf die Entscheidung über den Einspruch: **22.07.2026**
(45) Hinweis auf die Patenterteilung: 23.08.2023
(21) Anmeldenummer: 18785949.1
(22) Anmeldetag: 11.10.2018
(51) Int. Cl.: A24F 40/42, A24F 40/48, A24F 40/51, A61M 11/04, A61M 15/02, A61M 15/06

(54) **FLÜSSIGKEITSSPEICHER FÜR EINEN INHALATOR, INSBESONDERE FÜR EIN ELEKTRONISCHES ZIGARETTENPRODUKT**
LIQUID STORE FOR AN INHALER, IN PARTICULAR FOR AN ELECTRONIC CIGARETTE PRODUCT
CARTOUCHE DE LIQUIDE DESTINÉE À UN INHALATEUR, NOTAMMENT À UN PRODUIT CIGARETTE ÉLECTRONIQUE

(30) Priorität: 13.10.2017 DE 102017123869
(43) Veröffentlichungstag der Anmeldung: 19.08.2020
(73) Patentinhaber: Körber Technologies GmbH, 21033 Hamburg (DE)
(72) Erfinder: TRIEU, Hoc Khiem, 21394 Westergellersen (DE); KALAYDZHYAN, Karen, 22607 Hamburg (DE)
(74) Vertreter: Müller Verweyen
(86) Internationale Anmeldenummer: PCT/EP2018/077744
(87) Internationale Veröffentlichungsnummer: WO 2019/072971

(56) Entgegenhaltungen:
- EP-A1- 0 845 220
- WO-A1-2017/093535
- WO-A1-2018/083007
- DE-A1- 102014 114 133
- DE-U1- 202014 101 125

## Beschreibung

Die vorliegende Erfindung betrifft eine Verbrauchseinheit für einen Inhalator nach dem Oberbegriff von Anspruch 1.

Im Stand der Technik ist eine Vielzahl elektronischer Zigarettenprodukte bekannt, bei denen der Flüssigkeitsspeicher entweder als ein Reservoir bzw. Tank ausgeführt ist, oder bei denen die Flüssigkeit bzw. das Liquid in einem Schwamm beispielsweise aus Acetat gespeichert ist. In beiden Fällen kommuniziert das Reservoir mindestens über einen Docht und den Heizkörper, welcher in einem nach außen offenen Luftkanal angeordnet ist, mit der Außenwelt und ist mit dieser in einem Druckgleichgewicht.

Beide zuvor beschriebenen Varianten haben den Nachteil der Undichtigkeit. Zum einem kann Liquid bei starker Änderung des Umgebungsdrucks, beispielsweise in einem Flugzeug, austreten. Zum anderen kann der Nutzer bei nicht vorhandener Verdampferfunktion, beispielsweise wenn der Energiespeicher des elektronischen Zigarettenproduktes leer ist, das Liquid direkt durch einen Zug und den dadurch entstehenden Unterdruck aus dem Flüssigkeitsspeicher saugen. Dies ist unangenehm und führt zu Irritationen im Mundbereich.

Im Stand der Technik kommt es weiterhin zu teilweisem oder komplettem Trockenlaufen der Verdampfereinheit und zu nicht ausreichender Benetzung der Oberfläche des Verdampfers. In beiden Fällen entstehen durch die fehlende Kühlung durch das zu verdampfende Liquid Temperaturen oberhalb von 250 °C, was zur Entstehung von Schadstoffen durch Dekomposition und Radikalisierung der Liquidkomponenten, vor allem Glycerin und Propylenglycol, führt. Durch eine unkontrollierte bzw. nicht erfasste Zufuhr und Verdampfung von Liquid wird die Qualität des entstehenden Aerosols unbeabsichtigt und unkontrolliert beeinflusst.

Die DE 20 2014 101 125 U1 offenbart eine elektronische Zigarette mit einem Verdampfer zum Verdampfen eines Liquids. Das zu verdampfende Liquid gelangt über einen Liquidträger aus einem Liquidspeicher zu einem Heizelement. Der Liquidträger besteht dabei aus Glasfasern, Mineralfasern oder einem Sinterwerkstoff wie einer Keramik.

Aus der DE 10 2014 114 133 A1 ist eine elektrische Zigarette mit einem Verdampfer und einem mit dem Verdampfer verbundenem Depot für das Liquid vorgesehen. Das Depot kann beispielsweise als im Spritzgussverfahren hergestellter Kapillarspeicher mit Kapillaren ausgebildet sein.

Die EP 0 845 220 A1 offenbart ein aromaerzeugendes Gerät mit einem Materialbehälter zum Speichern einer Flüssigkeit enthaltend eine Aromasubstanz. Es ist ferner eine Abgabe-Antriebseinrichtung vorgesehen, welche zur Abgabe der Flüssigkeit durch eine Abgabeöffnung in Form eines Tropfens eingerichtet ist.

Die Aufgabe der Erfindung besteht darin, eine Verbrauchseinheit bereitzustellen, welche eine leckagefreie und sichere Speicherung, eine zuverlässige Nachförderung von Liquid und eine schwerkraftunabhängige Füllstandsmessung, unabhängig von der Orientierung des Flüssigkeitsspeichers während des Gebrauchs ermöglicht.

Die Erfindung löst diese Aufgabe mit den Merkmalen des unabhängigen Anspruchs.

Gemäß der Erfindung wird vorgeschlagen, dass der Flüssigkeitsspeicher wenigstens ein Lufteinlassende und wenigstens einen Kanal aufweist, und der Kanal sich von dem Lufteinlassende durch den Flüssigkeitsspeicher zu dem Auslassende erstreckt, wobei der Kanal dazu eingerichtet ist, die in dem Flüssigkeitsspeicher gespeicherte Flüssigkeit durch den Kanal mittels Kapillarkräften zu dem Auslassende zu fördern. Vorgeschlagen wird demnach ein insbesondere mikrofluidischer Flüssigkeitsspeicher, aus dem, über das gesamte Volumen des Reservoirs hinweg, Liquid mit gleichmäßiger und gravitations- und insbesondere orientierungsunabhängiger Rate, entweder aktiv oder passiv, kontrollierbar einem Heizkörper zuführbar ist. Der Kanal erstreckt sich durch den Flüssigkeitsspeicher und dient sowohl der Speicherung als auch der Förderung der Flüssigkeit. Der Liquidtransport durch das Reservoir bzw. den Kanal geschieht auf Basis von Kapillarkräften und/oder unter gezielter Nutzung des Laplace-Drucks. Kapillarkräfte entstehen, wenn an dem Auslassende Liquid entnommen wird, um dieses dem Heizkörper zuzuführen, und das verbleibende Liquid dem Druckgefälle folgt und am Lufteinlassende Luft zum Druckausgleich einströmt.

Die Erfindung erlaubt auf diese Weise eine schwerkraftunabhängige Füllstandsüberwachung des Flüssigkeitsspeichers. Die Schwerkraft ist typischerweise erheblich kleiner als die in dem erfindungsgemä-βen Flüssigkeitsspeicher auf das Liquid wirkende Kapillarkräfte.

Somit ist das in dem Flüssigkeitsspeicher befindliche Liquid schwerkraftunabhängig und insbesondere orientierungsunabhängig gehalten, was eine zuverlässige Füllstandsüberwachung erlaubt.

Des Weiteren sind die Kräfte, die ein Konsument durch einen Zug am Inhalator aufbringen kann, typischerweise erheblich kleiner als die Kapillarkräfte, die zu der Förderung des Liquids dienen und somit kann verhindert werden, dass der Konsument flüssiges Liquid aus der Verdampfereinheit zieht, ohne dass dieses verdampft wird.

Erfindungsgemäß weist der Flüssigkeitsspeicher einen insbesondere kapazitiven und/oder resistiven Füllstandssensor auf, der mindestens eine entlang des mindestens einen Kanals angeordnete Messelektrode aufweist. Ein kapazitiver Füllstandssensor entlang des mindestens einen Kanals ermöglicht die genaue und zuverlässige Bestimmung des Füllstandes der Flüssigkeit in dem Flüssigkeitsspeicher und/oder in dem wenigstens einen Kanal. Das in dem Flüssigkeitsspeicher befindliche Liquid dient dabei als Dielektrikum der kapazitiven Messung des Füllstandes. Die Kapazität ist durch das Dielektrikum bestimmt und somit eindeutig abhängig vom Füllstand der Flüssigkeit im Flüssigkeitsspeicher.

In einer bevorzugten Ausführungsform ist die Messelektrode von einem elektrisch leitfähigen Teil des Flüssigkeitsspeichers ausgebildet. Eine besonders kostengünstige und platzsparende Ausführungsform integriert die Elektrode in den Flüssigkeitsspeicher. Der wenigstens eine Kanal ist durch Kanalwände begrenzt und diese können die Elektrode aufweisen und/oder ausbilden. Die wenigstens eine Messelektrode kann beispielsweise streifenförmig sein oder durch wenigstens einen Messpunkt gegeben sein. Bevorzugt sind die Kanalwände elektrisch leitfähig, beispielsweise indem sie aus einem elektrisch leitfähigen Material bestehen oder eine elektrisch leitfähige Beschichtung aufweisen, und bilden die Messelektrode. Die Kanalwände können abschnittsweise, mindestens aber Auslassseitig, an einem oder mehreren Messpunkten und/oder Messstreifen eine kapazitive Messung des Liquidlevels ermöglichen.

Vorzugsweise ist an dem wenigstens einem Lufteinlassende eine semipermeable Dichtung und/oder Filterschicht angeordnet, wobei die semipermeable Dichtung und/oder Filterschicht luftdurchlässig und flüssigkeitsundurchlässig ist. Die semipermeable Dichtung und/oder Filterschicht wirkt der Leckage des Flüssigkeitsspeichers entgegen, indem der Durchtritt von Liquid durch das Lufteinlassende gehemmt oder unterbunden ist. Die Luftdurchlässigkeit der semipermeablen Dichtung und/oder Filterschicht ermöglicht einen Druckausgleich im Kanal, der bei der Entnahme von Liquid an dem Auslassende nötig ist, um eine kontinuierliche Liquidförderung zu gewährleisten und der Entstehung eines Unterdrucks vorzubeugen.

Es ist von Vorteil, dass der Flüssigkeitsspeicher eine Mehrzahl von Kanälen aufweist, um ein ausreichend großes Volumen zur Speicherung von Flüssigkeit bereitzustellen und kapillaren Transport von Liquid zu begünstigen. Das Volumen des Flüssigkeitsspeichers ist durch das Volumen des Kanals oder die Volumina der Kanäle gegeben. Die Kapillarkräfte können sich aus dem Querschnitt des Kanals oder den Querschnitten der Kanäle ergeben. Die Verwendung einer Mehrzahl von Kanälen im Flüssigkeitsspeicher kann daher dazu dienen, einen Flüssigkeitsspeicher mit einem ausreichend großen Gesamtvolumen bei gleichzeitig zuverlässiger Liquidförderung bereitzustellen. Die Mehrzahl von Kanälen kann in Form von Poren oder Löchern als Array angeordnet sein und/oder zylindrisch koaxial, ineinandergreifend oder abschnittsweise ineinandergreifend ausgebildet sein.

Vorzugsweise ist der mindestens eine Kanal zwischen mindestens zwei Gehäuseteile des Flüssigkeitsspeichers gebildet, die jeweils mindestens eine Kanalwand aufweisen, wobei die Kanalwände bei montierten Gehäuseteilen kammartig ineinandergreifen, um eine möglichst kostengünstige, einfache und effektive Montage zu gewährleisten und ein definierbares Volumen des Flüssigkeitsspeichers mit bei der Montage gezielt einstellbaren Kapillarkräften zu erzielen. Die kammartig ineinandergreifenden Kanalwände greifen derart ineinander, dass sich zwischen den Kanalwänden ein Volumen für das Liquid, also der wenigstens eine Kanal, bildet.

Die Geometrie der Gehäuseteile sowie deren Abstand im montierten Zustand bestimmen das Volumen und den Querschnitt des wenigstens einen Kanals und mithin die darin wirkenden Kapillarkräfte. Jeweils ein Gehäuseteil kann in dieser Ausführungsform eine Messelektrode aufweisen und/oder ausbilden, um eine einfache, zuverlässige und umfassende kapazitive und/oder resistive Messung des Füllstands des Flüssigkeitsspeichers zu ermöglichen.

In einer bevorzugten Ausführungsform bestehen die Gehäuseteile aus einem elektrisch leitenden Material oder sind mit einem elektrisch leitenden Material beschichtet, um eine möglichst effektive und einfache Integration von Elektroden zur Überwachung des Flüssigkeitsstandes zu ermöglichen. Die Gehäuseteile können beispielsweise aus einem Kunststoff hergestellt werden und anschließend wenigstens teilweise beschichtet und/oder bedampft werden, um elektrisch leitfähig zu sein.

In einer bevorzugten Ausführungsform weist der wenigstens eine Kanal Bereiche unterschiedlicher Hydrophilizität und/oder Hydrophobizität auf, um den Transport von Liquid durch den Kanal gezielt zu beeinflussen. D.h. Kanalwände, welche den wenigstens einen Kanal bilden, weisen Bereiche unterschiedlicher Hydrophilizität und/oder Hydrophobizität auf. Das Liquid hat wässrige Bestandteile und lässt sich gezielt durch die Bereiche unterschiedlicher Hydrophilizität und/oder Hydrophobizität steuern.

Vorzugsweise weist der wenigstens eine Kanal am Lufteinlassende eine niedrigere Hydrophilizität auf als am Auslassende, um ein Austritt von Liquid am Lufteinlassende zu hemmen und den Austritt von Liquid am Auslassende zu fördern. D.h., die Wände des Reservoirs, bzw. des wenigstens einen Kanals, sind hydrophober als die heizerseitige Auslassöffnung, um den Transport des wasserhaltigen Liquids in Richtung der Auslassöffnung zu begünstigen.

Bevorzugt ist der wenigstens eine Kanal am Lufteinlassende hydrophob, um einen ungewollten Austritt von wasserhaltigen Liquid am Lufteinlassende zu hemmen und/oder zu unterbinden. Das hydrophobe Lufteinlassende begünstigt die Dichtwirkung und Leckagefreiheit des Flüssigkeitsspeichers.

Es ist von Vorteil, dass der wenigstens eine Kanal am Auslassende hydrophil ist, um den Austritt am und/oder den Transport zum Auslassende von wasserhaltigen Liquid zu begünstigen. Am Auslassende kann das Liquid durch einen auf Kapillarkräften und somit passiv und/oder durch beispielsweise Pumpen und/oder Ventile und somit aktiv entnommen werden.

Die Erfindung betrifft eine Verbrauchseinheit, umfassend den Flüssigkeitsspeicher und eine Verdampfereinheit mit dem elektrisch betreibbaren Heizkörper, wobei der Heizkörper durch Anlegen einer Heizspannung zum Verdampfen von dem Heizkörper aus dem Flüssigkeitsspeicher zugeführter Flüssigkeit eingerichtet ist.

Bevorzugt ist eine Dochtstruktur zwischen dem Heizkörper und dem Flüssigkeitsspeicher angeordnet, wobei die Dochtstruktur dazu eingerichtet ist, Flüssigkeit aus dem wenigstens einem Auslassende des Flüssigkeitsspeichers aufzunehmen und mittels Kapillarkräften zum Heizkörper zu fördern. Der Docht dient der passiven Liquidentnahme an dem wenigstens einem Auslassende und dem Aufbau eines negativen Laplace-Drucks, welcher die Nachförderung von Liquid bedingt.

Vorzugsweise überdeckt die Dochtstruktur das oder sämtliche Auslassenden, um Leckagefreiheit und einen gleichmäßigen Transport von Liquid vom Flüssigkeitsspeicher über den Docht zu dem Heizkörper zu begünstigen.

Vorzugsweise ist an dem Heizkörper wenigstens ein kapazitiver und/oder resistiver Sensor zur Detektion von Flüssigkeit angeordnet, um den Betriebszustand des Heizkörpers zu charakterisieren und/oder die Anwesenheit und/oder Menge an zu verdampfender Flüssigkeit zu ermitteln. Durch einen beispielsweise metallischen Sensor in unmittelbarer Nähe des Heizkörpers, vorzugsweise direkt unterhalb eines flächig ausgebildeten Heizkörpers, weiter vorzugsweise mit direkten Kontakt zum Liquid, welches dem Heizkörper zugeführt wird und bevorzugt innerhalb des kapillaren Dochtmaterials, welches das Liquid dem Heizkörper zuführt, kann eine Liquiddetektion am Heizkörper stattfinden.

Erfindungsgemäß ist an dem Heizkörper wenigstens ein insbesondere resistiver Temperatursensor zur Messung der Temperatur des Heizkörpers und/oder der am Heizkörper vorbeiströmenden Luft eingerichtet, um den Betriebszustand in direkter Umgebung des Heizkörpers zu charakterisieren und/oder eine Überhitzung und/oder Unterkühlung des Heizkörpers bzw. des zu verdampfenden Liquids zu detektieren. Das zusätzliche Temperaturelement dient zur Überprüfung der Kühlfunktion der am Heizkörper vorbeiströmenden Umgebungsluft und damit direkt zur Messung der Lufttemperatur. Die Lufttemperatur hat einen Einfluss auf das Rekondensationsverhalten des Aerosols. Eine Kenntnis der Lufttemperatur erlaubt damit eine Vorhersage der wahrscheinlichen Tröpfchengrößen unter Berücksichtigung der am Verdampfer eingestellten Parameter, bzw. die Anpassung dieser Parameter zur Einflussnahme auf die Tröpfchengröße.

Um eine kompakte Ausführung des Temperatursensors zu ermöglichen, ist dieser vorzugsweise mäanderförmig ausgeführt. Es ist auch möglich, dass der Temperatursensor von dem Heizkörper gebildet ist.

Bevorzugt ist wenigstens einer der Sensoren als Leiterbahn auf einem Träger und weiter bevorzugt auf der gleichen Seite des Trägers wie der Heizkörper angeordnet, um eine effiziente Bauweise und eine zielgerichtete Messung zu ermöglichen.

Die Erfindung wird im Folgenden anhand bevorzugter Ausführungsformen unter Bezugnahme auf die beigefügten Figuren erläutert. Dabei zeigt
- Fig. 1: eine schematische Darstellung eines elektronischen Zigarettenprodukts;
- Fig. 2: eine schematische perspektivische Querschnittsansicht einer Verdampfereinheit mit einem erfindungsgemäßen Flüssigkeitsspeicher;
- Fign. 3, 4: schematische Darstellungen einer Ausführungsform eines Flüssigkeitsspeichers;
- Fign. 5, 6: schematische Darstellungen einer weiteren Ausführungsform eines Flüssigkeitsspeichers; und
- Fig. 7: einen Heizkörper auf einem Träger mit einem Sensor zur Detektion von Flüssigkeit und einem Temperatursensor.

Der Inhalator 10, hier ein elektronisches Zigarettenprodukt, umfasst ein Gehäuse 11, in dem ein Luftkanal 30 zwischen mindestens einer Lufteinlassöffnung 31 und einer Luftauslassöffnung 24 an einem Mundende 32 des Zigarettenprodukts 10 vorgesehen ist. Das Mundende 32 des Zigarettenprodukts 10 bezeichnet dabei das Ende, an dem der Konsument zwecks Inhalation zieht und dadurch das Zigarettenprodukt 10 mit einem Unterdruck beaufschlagt und eine Luftströmung 34 in dem Luftkanal 30 erzeugt.

Das Zigarettenprodukt 10 besteht vorteilhaft aus einem Basisteil 16 und einer Verbrauchseinheit 17, die die Verdampfereinheit 20 und den Flüssigkeitsspeicher 18 umfasst und insbesondere in Form einer auswechselbaren Kartusche ausgebildet ist. Die durch die Einlassöffnung 31 angesaugte Luft wird in dem Luftkanal 30 zu oder entlang mindestens einer Verdampfereinheit 20 geleitet. Die Verdampfereinheit 20 ist mit mindestens einem Flüssigkeitsspeicher 18 verbunden oder verbindbar, in dem mindestens eine Flüssigkeit 50 gespeichert ist. Die Verdampfereinheit 20 verdampft Flüssigkeit 50, die ihr aus dem Flüssigkeitsspeicher 18 zugeführt wird, und gibt die verdampfte Flüssigkeit als Aerosol/Dampf an einer Auslassseite 64 in den Luftstrom 34 zu. Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml.

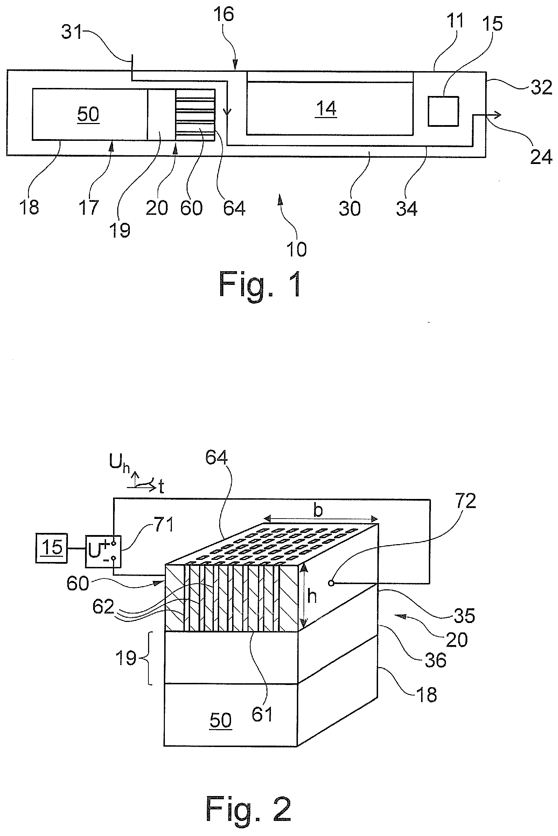

Die elektronische Zigarette 10 umfasst des Weiteren einen elektrischen Energiespeicher 14 und eine elektronische Steuerungsvorrichtung 15. Der Energiespeicher 14 ist in der Regel in dem Basisteil 16 angeordnet und kann insbesondere eine elektrochemische Einweg-Batterie oder ein wiederaufladbarer elektrochemischer Akku, beispielsweise ein Lithium-Ionen-Akku, sein. Die elektronische Steuerungsvorrichtung 15 umfasst mindestens eine digitale Datenverarbeitungseinrichtung, insbesondere Mikroprozessor und/oder Mikrocontroller, in dem Basisteil 16 (wie in Figur 1 gezeigt) und/oder in der Verbrauchseinheit 17.

In dem Gehäuse 11 ist vorteilhaft ein Sensor, beispielsweise ein Drucksensor oder ein Druck- oder Strömungsschalter, angeordnet, wobei die Steuerungsvorrichtung 15 auf der Grundlage eines von dem Sensor ausgegebenen Sensorsignals feststellen kann, dass ein Konsument am Mundende 32 des Zigarettenprodukts 10 zieht, um zu inhalieren. In diesem Fall steuert die Steuerungsvorrichtung 15 die Verdampfereinheit 20 an, um Flüssigkeit 50 aus dem Flüssigkeitsspeicher 18 als Aerosol/Dampf in den Luftstrom 34 zuzugeben.

Die in dem Flüssigkeitsspeicher 18 gespeicherte, zu dosierende Flüssigkeit 50 ist beispielsweise eine Mischung aus 1,2-Propylenglykol, Glycerin, Wasser, mindestens einem Aroma (Flavour) und/oder mindestens einem Wirkstoff insbesondere Nikotin.

Die Verbrauchseinheit bzw. Kartusche 17 umfasst vorteilhaft einen nichtflüchtigen Datenspeicher zum Speichern von die Verbrauchseinheit bzw. Kartusche 17 betreffender Information bzw. Parameter. Der Datenspeicher kann Teil der elektronischen Steuerungsvorrichtung 15 sein. In dem Datenspeicher ist vorteilhaft Information zur Zusammensetzung der in dem Flüssigkeitsspeicher 18 gespeicherten Flüssigkeit, Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, eine ID zur eindeutigen Kennzeichnung der Verbrauchseinheit bzw. Kartusche 17, Seriennummer, Herstelldatum und/oder Ablaufdatum, und/oder Zugzahl (Anzahl der Inhalationszüge durch den Konsumenten) bzw. der Nutzungszeit gespeichert. Der Datenspeicher ist vorteilhaft über Kontakte und/oder Leitungen mit der Steuereinrichtung 15 verbunden oder verbindbar.

Eine vorteilhafte Ausführungsform einer erfindungsgemäßen Verdampfereinheit 20 ist in Fig. 2 gezeigt. Die Verdampfereinheit 20 umfasst einen blockförmigen, vorzugsweise monolithischen Heizkörper 60 aus einem elektrisch leitenden Material, vorzugsweise Silizium, dotierte Keramik, Metall-Keramik, Filter-Keramik, Halbleiter, insbesondere Germanium, Graphit, Halbmetall und/oder Metall. Es ist nicht erforderlich, dass der gesamte Heizkörper 60 aus einem elektrisch leitenden Material besteht. Es kann beispielsweise ausreichen, dass die Oberfläche des Heizkörpers 60 elektrisch leitend, beispielsweise metallisch, beschichtet ist. In diesem Fall muss nicht die gesamte Oberfläche beschichtet sein, beispielsweise können Leiterbahnen auf einem nichtleitenden Grundkörper vorgesehen sein.

Der Heizkörper 60 ist mit einer Mehrzahl von Mikrokanälen 62 versehen, die eine Einlassseite 61 des Heizkörpers 60 mit einer Auslassseite 64 flüssigkeitsleitend verbinden. Die Einlassseite 61 ist über eine Dochtstruktur 19 flüssigkeitsleitend mit dem Flüssigkeitsspeicher 18 verbunden. Die Dochtstruktur 19 dient zur passiven Förderung von Flüssigkeit aus einem Flüssigkeitsspeicher 18 zu dem Heizkörper 60 mittels Kapillarkräften. Im Kontaktbereich 61 zu dem Heizkörper 60 dient die Dochtstruktur 19 dazu, Flüssigkeit gleichmäßig zu verteilen, temperaturbeständig zu sein und mit ihren relativ kleinen Poren und/oder dünnen Kapillaren eine Art Rückschlagventil zu bilden, um unerwünschtes Rückfließen von blasenhaltiger Flüssigkeit aus dem Heizkörper 60 in die Dochtstruktur 19 und/oder in den Flüssigkeitsspeicher 18 zu verhindern.

Der mittlere Durchmesser der Mikrokanäle 62 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 61 in einen Mikrokanal 62 eindringende Flüssigkeit durch den Mikrokanal 62 nach oben steigt, bis der Mikrokanal 62 mit Flüssigkeit gefüllt ist. Das Volumenverhältnis von Mikrokanälen 62 zu Heizkörper 60, das als Porosität des Heizkörpers 60 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen der mit Mikrokanälen 62 versehenen Flächen des Heizkörpers 60 liegen beispielsweise im Bereich zwischen 0,5 mm und 3 mm. Die Abmessungen der mit Mikrokanälen 62 versehenen Flächen des Heizkörpers 60 können beispielsweise betragen: 0,95 mm × 1,75 mm oder 1,9 mm × 1,75 mm oder 1,9 mm × 0,75 mm. Die Kantenlängen des Heizkörpers 60 können beispielsweise im Bereich zwischen 0,5 mm und 5 mm, vorzugsweise im Bereich zwischen 0,75 mm und 4 mm, weiter vorzugsweise im Bereich zwischen 1 mm und 3 mm liegen. Die Fläche des Heizkörpers 60 (chip size) kann beispielsweise 1 mm × 3 mm oder 2 mm × 3 mm betragen.

Die Breite b des Heizkörpers 60 (siehe Figur 2) liegt vorzugsweise im Bereich zwischen 1 mm und 5 mm, weiter vorzugsweise im Bereich zwischen 2 mm und 4 mm, und beträgt beispielsweise 3 mm. Die Höhe h des Heizkörpers 60 (siehe Figur 2) liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm.

Die Anzahl der Mikrokanäle 62 liegt vorzugsweise im Bereich zwischen vier und 1000. Auf diese Weise lässt sich der Wärmeeintrag in die Mikrokanäle 62 optimieren und eine gesicherte hohe Verdampfungsleistung sowie eine ausreichend große Dampfaustrittsfläche realisieren.

Die Mikrokanäle 62 sind in Form eines quadratischen, rechteckigen, vieleckigen, runden, ovalen oder anders geformten Arrays angeordnet. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Mikrokanäle 62 mit gesichert hoher Verdampfungsleistung realisieren.

Der Querschnitt der Mikrokanäle 62 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge eines oder jedes Mikrokanals 62 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 500 µm und beträgt beispielsweise 300 µm. Auf diese Weise lässt sich eine optimale Flüssigkeitsaufnahme und Portionsbildung bei ausreichend gutem Wärmeeintrag von dem Heizkörper 60 in die Mikrokanäle 62 realisieren.

Der Abstand zweier Mikrokanäle 62 beträgt vorzugsweise mindestens das 1,3-fache des lichten Durchmessers eines Mikrokanals 62, wobei der Abstand auf die Mittelachsen der beiden Mikrokanäle 62 bezogen ist. Der Abstand kann bevorzugt das 1,5- bis 5-fache, weiter bevorzugt das 2- bis 4-fache des lichten Durchmessers eines Mikrokanals 62 betragen. Auf diese Weise lässt sich ein optimaler Wärmeeintrag in die Mikrokanäle und eine ausreichend stabile Anordnung und Wandstärke der Mikrokanäle realisieren.

Die Verdampfereinheit 20 weist eine vorzugsweise von der Steuerungsvorrichtung 15 steuerbare Heizspannungsquelle 71 auf, die über Elektroden 72 an gegenüberliegenden Seiten des Heizkörpers 60 mit diesem verbunden ist, so dass eine von der Heizspannungsquelle 71 erzeugte elektrische Spannung Uh zu einem Stromfluss durch den Heizkörper 60 führt. Aufgrund des Ohm'schen Widerstands des elektrisch leitenden Heizkörpers 60 führt der Stromfluss zu einer Erhitzung des Heizkörpers 60 und daher zu einer Verdampfung von in den Mikrokanälen 62 enthaltener Flüssigkeit. Der Heizkörper 60 wirkt somit als Verdampfer. Auf diese Weise erzeugter Dampf/Aerosol entweicht zur Auslassseite 64 aus den Mikrokanälen 62 und wird der Luftströmung 34 beigemischt, siehe Figur 1. Genauer steuert bei Feststellung eines durch Ziehen des Konsumenten verursachten Luftstroms 34 durch den Luftkanal 30 die Steuerungsvorrichtung 15 die Heizspannungsquelle 71 an, wobei durch spontane Erhitzung die in den Mikrokanälen 62 befindliche Flüssigkeit in Form von Dampf/Aerosol aus den Mikrokanälen 62 getrieben wird.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt vorteilhaft zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt.

Vorzugsweise ist in dem Datenspeicher des Inhalators 10 eine dem verwendeten Flüssigkeitsgemisch angepasste Spannungskurve Uh(t) hinterlegt. Dies ermöglicht es, den Spannungsverlauf Uh(t) dem verwendeten Liquid angepasst vorzugeben, so dass sich die Heiztemperatur des Heizkörpers 60, und damit auch die Temperatur der kapillaren Mikrokanäle 62, gemäß der bekannten Verdampfungskinetik des jeweiligen Liquids zeitlich über den Verdampfungsvorgang steuern lässt, wodurch optimale Verdampfungsergebnisse erzielbar sind. Die Verdampfungstemperatur liegt vorzugsweise im Bereich zwischen 100°C und 400°C, weiter bevorzugt zwischen 150 C und 350°C, noch weiter bevorzugt zwischen 190 C und 290 C.

An der Einlassseite 61 des Heizkörpers 60 ist eine poröse und/oder kapillare, flüssigkeitsleitende Dochtstruktur 19 angeordnet. Die Dochtstruktur 19 kontaktiert die Einlassseite 61 des Heizkörpers 60 flächig und deckt sämtliche Mikrokanäle 62 einlassseitig ab, wie in den Figur 2 ersichtlich ist. An der dem Heizkörper 60 gegenüberliegenden Seite ist die Dochtstruktur flüssigkeitsleitend mit dem Flüssigkeitsspeicher 18 verbunden. Die in den Figuren 1 und 2 gezeigte direkte Anbindung des Flüssigkeitsspeichers 18 an die Dochtstruktur 19 ist nur beispielhaft zu verstehen. Insbesondere können eine Flüssigkeitsschnittstelle und/oder eine mehrere Flüssigkeitsleitungen zwischen Flüssigkeitsspeicher 18 und Dochtstruktur 19 vorgesehen sein. Der Flüssigkeitsspeicher 18 kann daher auch beabstandet von der Dochtstruktur 19 angeordnet sein. Der Flüssigkeitsspeicher 18 kann in seinen Abmessungen größer als die Dochtstruktur 19 sein. Die Dochtstruktur 19 kann beispielsweise in eine Öffnung eines Gehäuses des Flüssigkeitsspeichers 18 eingesetzt sein. Es kann auch eine Mehrzahl von Verdampfereinheiten 20 einem Flüssigkeitsspeicher 18 zugeordnet sein.

Die Dochtstruktur 19 besteht aus porösem und/oder kapillarem Material, das aufgrund von Kapillarkräften in der Lage ist, von dem Heizkörper 60 verdampfte Flüssigkeit in ausreichender Menge von dem Flüssigkeitsspeicher 18 zu dem Heizkörper 60 passiv nachzufördern, um ein Leerlaufen der Mikrokanäle 62 und sich daraus ergebende Probleme zu verhindern.

Die Dochtstruktur 19 besteht vorteilhaft aus einem nichtleitenden Material, um eine unerwünschte Erwärmung von Flüssigkeit in der Dochtstruktur 19 durch Stromfluss zu vermeiden. Falls die Dochtstruktur 19 aus einem leitenden Material besteht, was nicht ausgeschlossen ist, ist zwischen der Dochtstruktur 19 und dem Heizkörper 60 vorteilhaft eine Isolierschicht aus einem elektrisch und/oder thermisch isolierenden Material, beispielsweise Glas, Keramik oder Kunststoff, mit sich durch die Isolierschicht erstreckenden, mit den Mikrokanälen 62 korrespondierenden Durchgangsöffnungen vorgesehen.

Die Dochtstruktur 19 besteht vorteilhaft aus einem oder mehreren der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, oder einem Verbund von zwei oder mehr der vorgenannter Materialien. In einer vorteilhaften praktischen Ausführungsform kann die Dochtstruktur 19 mindestens ein Keramikfaserpapier und/oder eine poröse Keramik umfassen. Das Volumen der Dochtstruktur 19 liegt vorzugsweise im Bereich zwischen 1 mm³ und 10 mm³, weiter vorzugsweise im Bereich zwischen 2 mm³ und 8 mm³, noch weiter vorzugsweise im Bereich zwischen 3 mm³ und 7 mm³ und beträgt beispielsweise 5 mm³.

Die Dochtstruktur 19 kann generell einteilig oder mehrteilig sein.

Der Heizkörper 60 kann vorteilhaft aus Teilstücken eines Wafers mit Dünnfilmschichttechnologie hergestellt werden, welcher eine Schichtdicke von vorzugsweise kleiner oder gleich 1000 µm, weiter vorzugsweise kleiner oder gleich 750 µm, noch weiter vorzugsweise kleiner oder gleich 500 µm aufweist. Oberflächen des Heizkörpers 60 können vorteilhaft hydrophil sein. Die Auslassseite 64 des Heizkörpers 60 kann vorteilhaft mikrostrukturiert sein bzw. Mikroausnehmungen (micro grooves) aufweisen.

Die Verdampfereinheit 20 ist so eingestellt, dass eine Flüssigkeitsmenge vorzugsweise im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann die Verdampfereinheit 20 hinsichtlich der Flüssigkeits-/Dampfmenge pro Zug einstellbar sein.

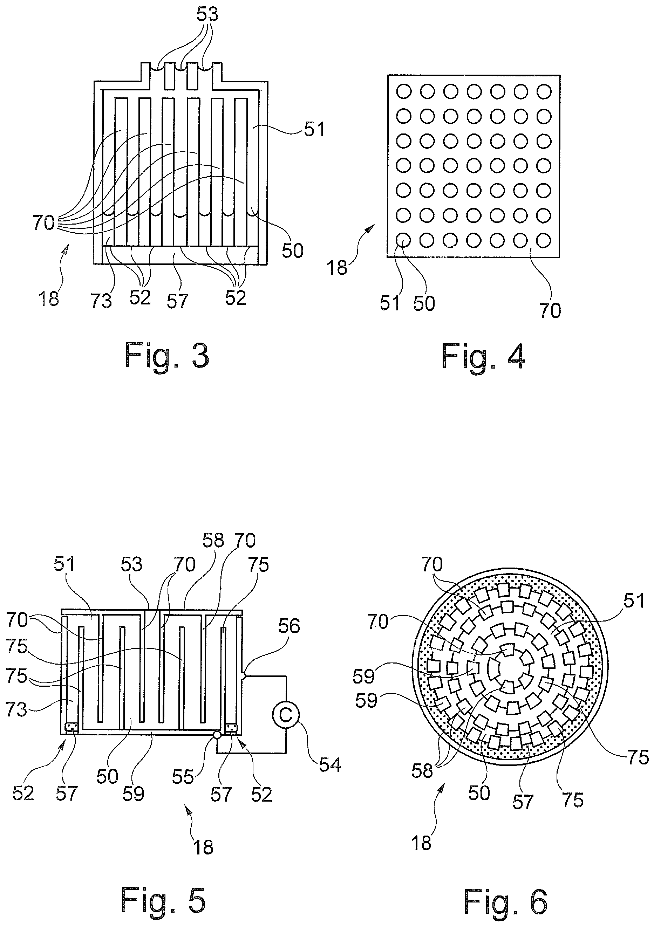

Figuren 3 und 4 zeigen Schnitte durch verschiedene Ebenen einer schematischen Darstellung einer Ausführungsform eines Flüssigkeitsspeichers 18, welcher einen rechteckigen Querschnitt aufweist. Der Flüssigkeitsspeicher 18 umfasst eine Mehrzahl von Auslassenden 53, eine Mehrzahl von Lufteinlassenden 52 sowie einer sich zwischen den Auslassenden 53 und Lufteinlassenden 52 erstreckenden Mehrzahl von Kanälen 51. Die Kanäle 51 sind durch Kanalwände 70, 75 voneinander getrennt und/oder werden durch die Kanalwände 70, 75 geführt bzw. gebildet. Die Mehrzahl von Lufteinlassenden 52 ist durch eine semipermeablen Dichtung und/oder Filterschicht 57 mit der Außenwelt gekoppelt. Die semipermeable Dichtung und/oder Filterschicht 57 erlaubt den Eintritt von Luft 73 durch die Lufteinlassenden 52 in den Kanal 51 und somit in den Flüssigkeitsspeicher 18. Die Anzahl und Geometrie der Auslassenden 53 ist an den jeweiligen Docht 19 angepasst.

Der Flüssigkeitsspeicher 18 enthält in den Kanälen 51 Liquid 50 und gegebenenfalls Luft 73, die aufgrund des Verbrauchs von Flüssigkeit 50 in den Flüssigkeitsspeicher eingedrungen ist. Vorzugsweise enthält der Flüssigkeitsspeicher 18 in einem werksseitig hergestellten Zustand ausschließlich Flüssigkeit 50 und keine Luft 73.

Mehrere Kanäle 51 können in nicht gezeigten Ausführungsformen in Form von Poren oder Löchern als Array angeordnet sein. Die Kanäle 51 können auch zylindrisch koaxial, ineinandergreifend oder abschnittsweise ineinandergreifend ausgebildet sein. Die Kanalwände 70, 75 können entsprechend einen runden, quadratischen, rechteckigen oder einen beliebigen anderen Querschnitt aufweisen. Der wenigstens eine Kanal 51 kann vorteilhaft gestreckt oder in beliebiger Form "aufgerollt", siehe Figur 6, oder gewunden angeordnet sein.

Figuren 5 und 6 zeigen Schnitte durch verschiedene Ebenen einer schematischen Darstellung einer weiteren Ausführungsform eines Flüssigkeitsspeichers 18 mit einem runden, vorzugsweise kreisförmigen, Querschnitt. In dieser Ausführungsform ist ein Auslassende 53 in der Mitte des kreisförmigen Querschnitts des Flüssigkeitsspeichers 18 angeordnet. In dieser Ausführungsform weist der Flüssigkeitsspeicher 18 ein Lufteinlassende 52 auf, welches ringförmig um den Umfang des kreisförmigen Flüssigkeitsspeichers 18 ausgebildet ist. Zwischen dem Auslassende 53 und dem Lufteinlassende 52 erstreckt sich ein Kanal 51. Die Geometrie des Kanals 50 ist durch Kanalwände 70, 75 bestimmt. Das Lufteinlassende 52 ist durch eine ringförmige semipermeable Dichtung und/oder Filterschicht 57 mit der Außenwelt gekoppelt. Die semipermeable Dichtung und/oder Filterschicht 57 erlaubt den Eintritt von Luft 73 durch das Lufteinlassende 52 in den Kanal 51 und somit in den Flüssigkeitsspeicher 18, hemmt aber den Austritt von Liquid 50 aus dem Flüssigkeitsspeicher 18.

In dieser Ausführungsform besteht der Flüssigkeitsspeicher 18 aus zwei Gehäuseteilen 58, 59. Die Gehäuseteile 58, 59 greifen kammartig ineinander, d.h. wie in Figur 5 zu sehen umfasst jedes der Gehäuseteile 58, 59 eine in dieser Ansicht oben bzw. unten angeordnete Basis und sich zu der Basis senkrecht erstreckende, von den Kanalwänden 70, 75 gebildete Zinken, sodass die Zinken der Gehäuseteilen 58, 59 einander entgegengerichtet angeordnet sind und wie zwei Kämme ineinandergreifen.

In Figur 6 ist verdeutlicht, dass das kammartige Ineinandergreifen 58, 59 zur Folge hat, dass die Kanalwände 70, 75 alternierend durch eines der Gehäuseteile 58, 59 gebildet werden. Ein Teil der Kanalwände 70 wird vorzugsweise von dem Gehäuseteil 58 gebildet und/oder ein Teil der Kanalwände 75 wird vorzugsweise von dem Gehäuseteil 59 gebildet. Der Kanal 51 wird somit durch zylindrisch koaxial, ineinander verflochtene beziehungsweise abschnittsweise ineinandergreifende Gehäuseteile 58, 59 gebildet. Zwischen den Gehäuseteilen 58, 59 bildet sich der Kanal 51, in denen die Flüssigkeit 50 und gegebenenfalls die Luft 73 gespeichert und/oder transportiert werden.

Weiterhin weist das in Figuren 5 und 6 gezeigte Ausführungsbeispiel des Flüssigkeitsspeichers 18 einen Füllstandssensor 54, vorzugsweise einen kapazitiven Füllstandssensor 54, auf. Der Füllstandssensor 54 ist mit wenigstens einer Messelektrode 55, 56 dazu eingerichtet, Füllstandsmessungen des Flüssigkeitsspeichers 18 auszuführen. In diesem Ausführungsbeispiel ist der Füllstandssensor 54 über einem Kontakt 55 mit dem Gehäuseteil 59 und über einen weiteren Kontakt 56 mit dem Gehäuseteil 58 verbunden. Vorteilhaft ist daher der Füllstandssensor 54 zwischen die als Elektroden wirkenden Gehäuseteile 58, 59 geschaltet. Vorzugsweise sind die Gehäuseteile 58, 59 aus einem elektrisch leitenden Material, um als Elektroden für die insbesondere kapazitive und/oder resistive Messung des Füllstandssensors 54 zu dienen. Das zwischen den Gehäuseteilen 58, 59 in dem Kanal 51 befindliche Liquid 50 sowie die gegebenenfalls in dem Kanal 51 befindliche Luft 73 stellen für die kapazitive Messung ein Dielektrikum dar. Das Liquid 50 und die Luft 73 weisen unterschiedliche Dielektrizitätskonstanten auf und bewirken somit eine füllstandsabhängige elektrische Kapazitätsänderung des Flüssigkeitsspeichers 18.

Die Entnahme von Flüssigkeit 50 an der heizerseitigen Öffnung, d.h. an dem wenigstens einem Auslassende 53, kann aktiv oder passiv geschehen. Eine passive Entnahme meint die Entnahme von Flüssigkeit 50 aus dem Flüssigkeitsspeicher 18 allein aufgrund von thermodynamischen und/oder hydrodynamischen Vorgängen, insbesondere Kapillarkräften und/oder Laplace-Druck. Eine aktive Entnahme meint die Entnahme von Flüssigkeit 50 aus dem Flüssigkeitsspeicher 18, die durch aktive, insbesondere elektromechanische Komponenten unterstützt ist.

Die Entnahme kann aktiv durch die Verdampfereinheit 20 gesteuert werden und/oder andere Elemente wie Ventile und Pumpen können für eine aktive Entnahme von Flüssigkeit 50 aus dem Flüssigkeitsspeicher 18 eingerichtet sein. Durch die aktive und/oder passive Entnahme von Liquid 50 erfolgt der Aufbau eines negativen Laplace-Drucks. Der negative Laplace-Druck kann auslassseitig, also nahe des Auslassendes 53, durch einen hydrophilen Wandabschnitt einer Kanalwand 70, 75 oder einen kapillar wirkenden Docht 19 oder ein beliebiges poröses Material aufgebaut werden.

Das dem Heizkörper 60 abgewandte Ende, d.h. das wenigstens eine Lufteinlassende 52, ist mit der semipermeablen Dichtung 57 versehen, die vorzugsweise Luft 73 in den Flüssigkeitsspeicher 18 eindringen, aber kein Liquid 50 aus dem Flüssigkeitsspeicher 18 entweichen lässt. Die semipermeable Dichtung 57 kann zum Beispiel ein hydrophober Schwamm oder eine poröse Keramik sein.

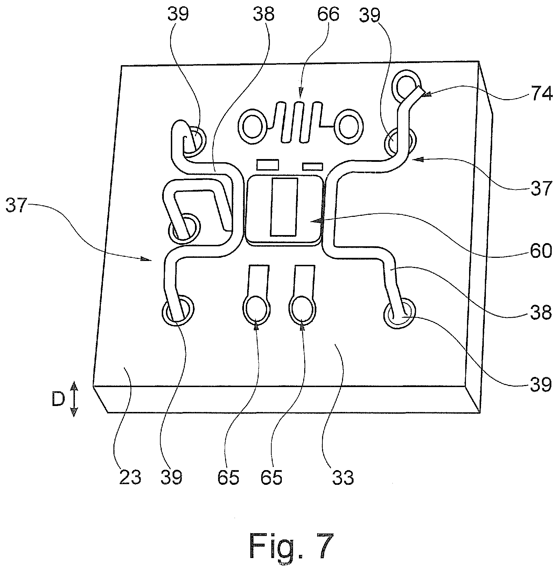

Wie in Figur 7 gezeigt ist, weist die Verdampfereinheit 20 vorteilhaft einen insbesondere plattenförmigen Träger 23 zum Halten des Heizkörpers 60 auf. Der Träger 23 kann aus einem geeigneten Material, beispielsweise Keramik, Glas und/oder Kunststoff einschließlich faserverstärktem Kunststoff, beispielsweise Leiterplattenmaterial bestehen und weist eine in Figur 7 nicht ersichtliche Durchgangsöffnung unter dem Heizkörper 60 auf, durch die sich die Dochtstruktur 19 in Richtung zu dem Flüssigkeitsspeicher 18 nach unten erstreckt.

Die Abmessungen des beispielsweise rechteckigen Trägers 23 liegen vorzugsweise im Bereich zwischen 6 mm und 20 mm, weiter vorzugsweise im Bereich zwischen 8 mm bis 17 mm und noch weiter vorzugsweise im Bereich zwischen 10 mm und 14 mm. Die Dicke D des Trägers 23 liegt vorzugsweise im Bereich zwischen 0,5 mm bis 4 mm, weiter vorzugsweise im Bereich zwischen 1 mm bis 3 mm, noch weiter vorzugsweise im Bereich zwischen 1 mm und 2 mm und kann beispielsweise 1,6 mm oder 2 mm betragen.

Die Klemmung des Heizkörpers 60 auf dem Träger 23 wird mittels mindestens zwei Klemmelementen 37 bewirkt, die an gegenüberliegenden Seiten des Heizkörpers 60 an diesem angreifen. Jedes Klemmelement 37 weist vorteilhaft einen Klemmbügel 38 auf, der an zwei voneinander beabstandeten Befestigungspunkten 39 federnd an dem Träger 23 befestigt ist und eine Vorspannung erzeugt, mittels der der Heizkörper 60 auf dem Träger 23 festgeklemmt und somit sicher gehalten wird.

Besonders vorteilhaft dienen die Klemmelemente 37 gleichzeitig als Elektroden zur Kontaktierung des Heizkörpers 60 und dessen Versorgung mit Heizstrom. Zu diesem Zweck bestehen die Klemmelemente 37 bzw. die Klemmbügel 38 vorteilhaft aus einem elektrisch leitenden Material, beispielsweise kann es sich um Metalldraht, beispielsweise Messingdraht handeln. Aufgrund der Linienkontaktierung zwischen dem Klemmbügel 38 und dem Heizkörper 60 ergibt sich eine ausgezeichnete elektrische Verbindung zwischen dem Klemmelement 37 und dem Heizkörper 60, bei gleichzeitig idealer thermischer Entkopplung zwischen dem Klemmelement 37 und dem Heizkörper 60 wegen fehlendem Flächenkontakt. Wärmedissipation von dem Heizkörper 60 in das Klemmelement 37 ist daher gering, die Elektroden 38 bleiben signifikant kühler als der Heizkörper 60.

Die Klemmelemente 37 können über Bohrungen in dem Träger 23 nach unten kontaktiert und mit einer in der Verbrauchseinheit 17 vorgesehenen Leiterplatte (PCB) verbunden sein, um die elektrische Verbindung zu der elektronischen Steuerungsvorrichtung 15 und zu der Energiequelle 14 für die Stromversorgung des Heizkörpers 60 herzustellen. In einer anderen Ausführungsform kann der Träger 23 die Leiterplatte ausbilden. Es ist auch möglich, dass die Verdampfereinheit 20 selbst keine Leiterplatte umfasst, sondern die Klemmbügel 38 beispielsweise über flexible isolierte Leitungen, oder auf andere geeignete Weise, mit einer etwa in dem Basisteil 16 angeordneten Leiterplatte verbunden sind.

Figur 7 zeigt einen Sensor 65 zur Detektion von Flüssigkeit und einen ebenfalls auf dem Träger 23 angeordneten Temperatursensor 66. Der Sensor 65 und der Sensor 66 sind an oder auf dem Träger 23 angeordnet. Der Sensor 65 und/oder der Sensor 66 ist bzw. sind vorteilhaft auf der gleichen Seite 33 des Trägers angeordnet wie die Auslassseite 64 des Heizkörpers.

In diesem Ausführungsbeispiel ist der Sensor 65 ein kapazitiver Flüssigkeitssensor 65 mit komplanaren Elektroden, d.h. die Elektroden sind in einer Ebene auf der Oberseite 33 des Trägers 23 angeordnet. Der insbesondere resistive Temperatursensor 66 ist hier mäanderförmig und als eine Leiterbahn ausgebildet, welche direkt auf dem Träger 23 aufgebracht, beispielsweise aufgedampft, ist. Auch die Elektroden 65 sind vorteilhaft als Leiterbahnen auf der Oberseite 33 des Trägers 23, der besonders vorteilhaft eine Leiterplatte (PCB) ist, aufgebracht.

Des Weiteren kann eine Sicherheitsvorrichtung in Form von einem Überhitzungsschutz 74 zum Schutz des Heizkörpers 60, des Liquids 50 und/oder empfindlicher elektronischer Bauteile vorgesehen sein. Der Überhitzungsschutz 74 kann beispielsweise einen BimetallStreifen umfassen. Der Überhitzungsschutz 74 kann Bestandteil eines Sicherheitssystems sein, welcher Sensoren und Aktoren umfasst und der die Anwesenheit von Flüssigkeit 50 auf dem Heizkörper 60 detektiert und die Temperatur in der Verdampfereinheit 20 misst und entsprechende Sicherheitsvorgänge steuert, welche beispielsweise Rückkopplungsschleifen unter Einschluss der Heizspannungsquelle 71 umfassen.

Durch die beschriebenen Sensoren 65, 66 ist eine Sicherheitsfunktion gegeben, die die direkte Umgebung des Heizkörpers 60 überwacht. Im Falle einer Überhitzung des Liquids 50 und/oder des Heizkörpers 60, zum Beispiel durch Missbrauch oder Fehlfunktion, kann das System abgeschaltet werden oder es können Gegenmaßnahmen zur Kühlung eingeleitet werden.

Meldet der kapazitive und/oder resistive Sensor 65 eine nicht ausreichende Menge von Liquid 50 am Heizer 60, was zu einer unerwünschten Erhöhung der Temperatur des Heizkörpers 60 führen kann, kann das System zum Beispiel in einen Sicherheitsmodus schalten und mit verminderter Leistung heizen oder den Heizkörper 60 abschalten.

Eine exakte Temperaturüberwachung in der direkten Umgebung des Heizkörpers 60 kann als zusätzliche Sicherheitsfunktion integriert sein, wenn beispielsweise der Heizkörper 60 als Temperaturmesselement eingerichtet ist, wie es etwa bei einem Silizium-Heizkörper 60 der Fall sein kann.

Weiterhin kann die Aerosolqualität durch die Menge an Liquid 50, welche dem Heizkörper 60 während eines Verdampfungsvorgangs zugeführt wird, d.h. durch den Liquidmassenstrom, beeinflusst werden. Die Aerosolqualität hat einen Einfluss auf das Rekondensationsverhalten des Aerosols. Eine Kenntnis über den Liquidmassenstrom und die zugrunde liegenden Zusammenhänge erlaubt damit eine Vorhersage der wahrscheinlichen Tröpfchengrößen unter Berücksichtigung der am Verdampfer 20 eingestellten Parameter, bzw. die Anpassung dieser Parameter zur Einflussnahme auf die Tröpfchengrößen. Der Liquidmassenstrom kann vorteilhaft mittels des kapazitiven und/oder resistiven Sensors 65 ermittelbar sein.

Die von der Heizspannungsquelle 71 erzeugte Ansteuerfrequenz des Heizkörpers 60 liegt vorteilhaft im Bereich von 1 Hz bis 50 kHz, bevorzugt im Bereich von 30 Hz bis 30 kHz, noch weiter vorteilhaft im Bereich von 100 Hz bis 25 kHz.

Im Folgenden wird der Ablauf des Verdampfungsvorgangs erläutert.

In einem Auslasszustand ist die Spannungsquelle 71 für den Heizvorgang ausgeschaltet.

Zum Verdampfen von Flüssigkeit 50 wird die Spannungsquelle 71 für den Heizkörper 60 aktiviert. Die Spannung Uh wird dabei so eingestellt, dass die Verdampfungstemperatur in dem Heizkörper 60 und somit in den Mikrokanälen 62 an das individuelle Verdampfungsverhalten des eingesetzten Flüssigkeitsgemischs angepasst ist. Dies verhindert die Gefahr von lokaler Überhitzung und dadurch Schadstoffentstehung.

Sobald eine Flüssigkeitsmenge verdampft ist, die dem Volumen der Mikrokanäle 62 entspricht oder damit in Zusammenhang steht, wird die Heizspannungsquelle 71 deaktiviert. Da die Liquideigenschaften und -menge vorteilhaft exakt bekannt sind, kann dieser Zeitpunkt sehr genau gesteuert werden. Die Energieaufnahme der Verdampfereinheit 20 lässt sich daher gegenüber bekannten Vorrichtungen reduzieren, da die benötigte Verdampfungsenergie dosierter und damit exakter eingebracht werden kann.

Nach Abschluss des Heizvorgangs sind die Mikrokanäle 62 überwiegend oder vollständig entleert. Die Heizspannung 71 wird dann so lange ausgeschaltet gehalten, bis mittels Nachförderung von Flüssigkeit 50 aus dem Flüssigkeitsspeicher 18 durch die Dochtstruktur 19 die Mikrokanäle 62 wieder aufgefüllt sind.

Über die Auslassenden 53 wird Flüssigkeit 50 aus dem Flüssigkeitsspeicher 18 zu dem Heizkörper 60, vorzugsweise über den Docht 19, nach gefördert. Durch die Entnahme von Flüssigkeit 50 aus dem Flüssigkeitsspeicher 18 leert sich der Flüssigkeitsspeicher 18 und ein dem Volumen der entnommenen Flüssigkeit 50 entsprechendes Volumen an Luft 73 wird über das wenigstens eine Lufteinlassende 52 durch die semipermeable Dichtung und/oder Filterschicht 57 in den Kanal 51 eingesaugt.

Sobald die Mikrokanälen 62 wieder aufgefüllt sind, kann der nächste Heizzyklus durch einschalten der Heizspannung 71 begonnen werden.

Die Verdampfereinheit 20 ist vorzugsweise auf der Grundlage von MEMS-Technologie, insbesondere aus Silizium, gefertigt und daher vorteilhaft ein Mikro-Elektro-Mechanisches System.

## Patentansprüche

1. Verbrauchseinheit (17) für einen Inhalator (10), umfassend eine Verdampfereinheit (20) mit einem elektrisch betreibbaren Heizkörper (60) und einen Flüssigkeitsspeicher mit einem Auslassende (53), das flüssigkeitsleitend mit dem Heizkörper (60) zum Verdampfen von dem Heizkörper (60) aus dem Flüssigkeitsspeicher (18) zugeführter Flüssigkeit (50) verbindbar ist, wobei der Heizkörper (60) durch Anlegen einer Heizspannung zum Verdampfen von dem Heizkörper (60) aus dem Flüssigkeitsspeicher (18) zugeführter Flüssigkeit (50) eingerichtet ist, wobei der Flüssigkeitsspeicher (18) wenigstens ein Lufteinlassende (52) und wenigstens einen Kanal (51) aufweist, und der Kanal (51) sich von dem Lufteinlassende (52) durch den Flüssigkeitsspeicher (18) zu dem Auslassende (53) erstreckt, wobei der Kanal (51) dazu eingerichtet ist, die in dem Flüssigkeitsspeicher (18) gespeicherte Flüssigkeit (50) durch den Kanal (51) mittels Kapillarkräften zu dem Auslassende (53) zu fördern, **dadurch gekennzeichnet, dass** der Flüssigkeitsspeicher (18) einen insbesondere kapazitiven und/oder resistiven Füllstandssensor (54) aufweist, der mindestens eine entlang des mindestens einen Kanals (51) angeordnete Messelektrode (55, 56) aufweist, wobei an dem Heizkörper (60) wenigstens ein insbesondere resistiver Temperatursensor (66) zur Messung der Temperatur des Heizkörpers (60) und/oder der am Heizkörper (60) vorbeiströmenden Luft (34) eingerichtet ist.

2. Verbrauchseinheit nach Anspruch 1, **dadurch gekennzeichnet, dass** die Messelektrode (55, 56) von einem elektrisch leitfähigen Teil des Flüssigkeitsspeichers (18) ausgebildet ist.

3. Verbrauchseinheit nach einem der vorangehenden Ansprüche, **dadurch gekennzeichnet, dass** an dem wenigstens einen Lufteinlassende (52) eine semipermeable Dichtung und/oder Filterschicht (57) angeordnet ist, wobei die semipermeable Dichtung und/oder Filterschicht (57) luftdurchlässig und flüssigkeitsundurchlässig ist.

4. Verbrauchseinheit nach einem der vorangehenden Ansprüche, **dadurch gekennzeichnet, dass** der Flüssigkeitsspeicher (18) eine Mehrzahl von Kanälen (51) aufweist.

5. Verbrauchseinheit nach einem der vorangehenden Ansprüche, **dadurch gekennzeichnet, dass** der mindestens eine Kanal (51) zwischen mindestens zwei Gehäuseteilen (58, 59) des Flüssigkeitsspeichers (18) gebildet ist, die jeweils mindestens eine Kanalwand (70, 75) aufweisen, wobei die Kanalwände (70, 75) bei montierten Gehäuseteilen (58, 59) kammartig ineinandergreifen.

6. Verbrauchseinheit nach Anspruch 5, **dadurch gekennzeichnet, dass** die Gehäuseteile (58, 59) aus einem elektrisch leitenden Material bestehen oder mit einem elektrisch leitenden Material beschichtet sind.

7. Verbrauchseinheit nach einem der vorangehenden Ansprüche, **dadurch gekennzeichnet, dass** der wenigstens eine Kanal (51) Bereiche unterschiedlicher Hydrophilizität und/oder Hydrophobizität aufweisen.

8. Verbrauchseinheit nach Anspruch 7, **dadurch gekennzeichnet, dass** der wenigstens eine Kanal (51) am Lufteinlassende (52) eine niedrigere Hydrophilizität aufweist als am Auslassende (53).

9. Verbrauchseinheit nach Anspruch 7 oder 8, **dadurch gekennzeichnet, dass** der wenigstens eine Kanal (51) am Lufteinlassende (52) hydrophob ist.

10. Verbrauchseinheit nach einem der Ansprüche 7 bis 9, **dadurch gekennzeichnet, dass** der wenigstens eine Kanal (51) am Auslassende (53) hydrophil ist.

11. Verbrauchseinheit nach einem der vorangehenden Ansprüche, **dadurch gekennzeichnet, dass** eine Dochtstruktur (19) zwischen dem Heizkörper (60) und dem Flüssigkeitsspeicher (18) angeordnet ist, wobei die Dochtstruktur (19) dazu eingerichtet ist, Flüssigkeit (50) aus dem wenigstens einem Auslassende (53) des Flüssigkeitsspeichers (18) aufzunehmen und mittels Kapillarkräften zum Heizkörper (60) zu fördern.

12. Verbrauchseinheit nach Anspruch 11, **dadurch gekennzeichnet, dass** die Dochtstruktur (19) das oder sämtliche Auslassenden (53) überdeckt.

13. Verbrauchseinheit nach einem der vorangehenden Ansprüche, **dadurch gekennzeichnet, dass** an dem Heizkörper (60) wenigstens ein insbesondere kapazitiver und/oder resistiver Sensor (65) zur Detektion von Flüssigkeit angeordnet ist.

14. Verbrauchseinheit nach Anspruch 13, **dadurch gekennzeichnet, dass** der Temperatursensor (66) mäanderförmig ist.

15. Verbrauchseinheit nach Anspruch 13, **dadurch gekennzeichnet, dass** der Temperatursensor (66) von dem Heizkörper (60) gebildet ist.

16. Verbrauchseinheit nach einem der Ansprüche 12 bis 15, **dadurch gekennzeichnet, dass** wenigstens einer der Sensoren (65, 66) als Leiterbahn auf einem Träger (23) für den Heizkörper (60) ausgebildet ist.

17. Verbrauchseinheit nach Anspruch 16, **dadurch gekennzeichnet, dass** wenigstens einer der Sensoren (65, 66) auf der gleichen Seite (33) des Trägers (23) angeordnet ist wie der Heizkörper (60).

## Claims

1. Consumption unit (17) for an inhaler (10), comprising a vaporizer unit (20) having an electrically operable heating body (60) and comprising a liquid reservoir having an outlet end (53) which is connectable in a liquid-conducting manner to the heating body (60) for vaporizing liquid (50) supplied to the heating body (60) from the liquid reservoir (18), wherein the heating body (60) is adapted to vaporize liquid (50) supplied to the heating body (60) from the liquid reservoir (18) by applying a heating voltage, wherein the liquid reservoir (18) comprises at least one air inlet end (52) and at least one channel (51), and the channel (51) extends from the air inlet end (52) through the liquid reservoir (18) to the outlet end (53), wherein the channel (51) is adapted to feed the liquid (50) stored in the liquid reservoir (18) through the channel (51) by means of capillary forces to the outlet end (53), **characterized in that** the liquid reservoir (18) comprises a level sensor (54), in particular a capacitive and/or resistive level sensor, which comprises at least one measuring electrode (55, 56) arranged along the at least one channel (51), wherein at least one, in particular resistive, temperature sensor (66) on the heating body (60) is adapted to measure the temperature of the heating body (60) and/or of the air (34) flowing past the heating body (60).

2. Consumption unit according to claim 1, **characterized in that** the measuring electrode (55, 56) is formed by an electrically conductive part of the liquid reservoir (18).

3. Consumption unit according to any one of the preceding claims, **characterized in that** a semipermeable seal and/or filter layer (57) is arranged at the at least one air inlet end (52), wherein the semipermeable seal and/or filter layer (57) is air-permeable and liquid-impermeable.

4. Consumption unit according to any one of the preceding claims, **characterized in that** the liquid reservoir (18) comprises a plurality of channels (51).

5. Consumption unit according to any one of the preceding claims, **characterized in that** the at least one channel (51) is formed between at least two housing parts (58, 59) of the liquid reservoir (18), which each comprise at least one channel wall (70, 75), wherein the channel walls (70, 75) interlock in a comb-like manner when the housing parts (58, 59) are assembled.

6. Consumption unit according to claim 5, **characterized in that** the housing parts (58, 59) consist of an electrically conductive material or are coated with an electrically conductive material.

7. Consumption unit according to any one of the preceding claims, **characterized in that** the at least one channel (51) comprises regions of different hydrophilicity and/or hydrophobicity.

8. Consumption unit according to claim 7, **characterized in that** the at least one channel (51) comprises a lower hydrophilicity at the air inlet end (52) than at the outlet end (53).

9. Consumption unit according to claim 7 or 8, **characterized in that** the at least one channel (51) is hydrophobic at the air inlet end (52).

10. Consumption unit according to any one of claims 7 to 9, **characterized in that** the at least one channel (51) is hydrophilic at the outlet end (53).

11. Consumption unit according to any one of the preceding claims, **characterized in that** a wick structure (19) is arranged between the heating body (60) and the liquid reservoir (18), wherein the wick structure (19) is adapted to receive liquid (50) from the at least one outlet end (53) of the liquid reservoir (18) and to feed it to the heating body (60) by means of capillary forces.

12. Consumption unit according to claim 11, **characterized in that** the wick structure (19) covers the or all outlet ends (53).

13. Consumption unit according to any one of the preceding claims, **characterized in that** at least one sensor (65), in particular a capacitive and/or resistive sensor, for detecting liquid is arranged on the heating body (60).

14. Consumption unit according to claim 13, **characterized in that** the temperature sensor (66) is meander-shaped.

15. Consumption unit according to claim 13, **characterized in that** the temperature sensor (66) is formed by the heating body (60).

16. Consumption unit according to any one of claims 12 to 15, **characterized in that** at least one of the sensors (65, 66) is formed as a conductor path on a carrier (23) for the heating body (60).

17. Consumption unit according to claim 16, **characterized in that** at least one of the sensors (65, 66) is arranged on the same side (33) of the carrier (23) as the heating body (60).

## Revendications

1. Unité de consommation (17) pour un inhalateur (10), comprenant une unité de vaporisation (20) pourvue d'un corps chauffant (60) à commande électrique et un réservoir de liquide pourvu d'une extrémité de sortie (53), qui peut être reliée avec conduction de liquide au corps chauffant (60) en vue de la vaporisation par le corps chauffant (60) du liquide (50) acheminé depuis le réservoir de liquide (18), le corps chauffant (60) étant conçu pour, par application d'une tension de chauffage, la vaporisation par le corps chauffant (60) du liquide (50) acheminé depuis le réservoir de liquide (18), le réservoir de liquide (18) possédant au moins une extrémité d'entrée d'air (52) et au moins un canal (51), et le canal (51) s'étendant de l'extrémité d'entrée d'air (52) à travers le réservoir de liquide (18) jusqu'à l'extrémité de sortie (53), le canal (51) étant conçu pour transporter le liquide (50) stocké dans le réservoir de liquide (18) à travers le canal (51) vers l'extrémité de sortie (53) par force capillaire, **caractérisée en ce que** le réservoir de liquide (18) possède un capteur de niveau (54), notamment capacitif et/ou résistif, qui possède au moins une électrode de mesure (55, 56) disposée le long de l'au moins un canal (51), au moins un capteur de température (66), notamment résistif, étant prévu sur le corps chauffant (60) pour mesurer la température du corps chauffant (60) et/ou de l'air (34) qui s'écoule le long du corps chauffant (60).

2. Unité de consommation selon la revendication 1, **caractérisée en ce que** l'électrode de mesure (55, 56) est formée par une partie électriquement conductrice du réservoir de liquide (18).

3. Unité de consommation selon l'une des revendications précédentes, **caractérisée en ce qu'**une garniture d'étanchéité semi-perméable et/ou une couche filtrante (57) est disposée au niveau de l'au moins une extrémité d'entrée d'air (52), la garniture d'étanchéité semi-perméable et/ou la couche filtrante (57) étant perméable à l'air et imperméable aux liquides.

4. Unité de consommation selon l'une des revendications précédentes, **caractérisée en ce que** le réservoir de liquide (18) possède une pluralité de canaux (51).

5. Unité de consommation selon l'une des revendications précédentes, **caractérisée en ce que** l'au moins un canal (51) est formé entre au moins deux parties de boîtier (58, 59) du réservoir de liquide (18), qui possèdent chacune au moins une paroi de canal (70, 75), les parois de canal (70, 75) s'emboîtant les unes dans les autres à la manière d'un peigne lorsque les parties de boîtier (58, 59) sont assemblées.

6. Unité de consommation selon la revendication 5, **caractérisée en ce que** les parties de boîtier (58, 59) sont constituées d'un matériau électriquement conducteur ou sont enduites d'un matériau électriquement conducteur.

7. Unité de consommation selon l'une des revendications précédentes, **caractérisée en ce que** l'au moins un canal (51) présente des zones ayant des hydrophilies et/ou des hydrophobies différentes.

8. Unité de consommation selon la revendication 7, **caractérisée en ce que** l'au moins un canal (51) présente une hydrophilie plus faible à l'extrémité d'entrée d'air (52) qu'à l'extrémité de sortie (53).

9. Unité de consommation selon la revendication 7 ou 8, **caractérisée en ce que** l'au moins un canal (51) est hydrophobe à l'extrémité d'entrée d'air (52).

10. Unité de consommation selon l'une des revendications 7 à 9, **caractérisée en ce que** l'au moins un canal (51) est hydrophile à l'extrémité de sortie (53).

11. Unité de consommation selon l'une des revendications précédentes, **caractérisée en ce qu'**une structure de mèche (19) est disposée entre le corps chauffant (60) et le réservoir de liquide (18), la structure de mèche (19) étant conçue pour recueillir du liquide (50) en provenance de l'au moins une extrémité de sortie (53) du réservoir de liquide (18) et le transporter vers le corps chauffant (60) par force capillaire.

12. Unité consommable selon la revendication 11, **caractérisée en ce que** la structure de mèche (19) recouvre la ou toutes les extrémités de sortie (53).

13. Unité de consommation selon l'une des revendications précédentes, **caractérisée en ce qu'**au moins un capteur (65), notamment capacitif et/ou résistif, destiné à détecter du liquide, est disposé sur le corps chauffant (60).

14. Unité consommable selon la revendication 13, **caractérisée en ce que** le capteur de température (66) est en forme de méandres.

15. Unité consommable selon la revendication 13, **caractérisée en ce que** le capteur de température (66) est formé par l'élément chauffant (60).

16. Unité consommable selon l'une des revendications 12 à 15, **caractérisée en ce qu'**au moins l'un des capteurs (65, 66) est réalisé sous forme de piste conductrice sur un support (23) de l'élément chauffant (60).

17. Unité consommable selon la revendication 16, **caractérisée en ce qu'**au moins l'un des capteurs (65, 66) est disposé du même côté (33) du support (23) que l'élément chauffant (60).
